(19) Europäisches Patentamt
European Patent Office
Office européen des brevets

(11) Veröffentlichungsnummer: **0 054 872**
**B1**

(12) # EUROPÄISCHE PATENTSCHRIFT

(45) Veröffentlichungstag der Patentschrift: **31.10.84**

(51) Int. Cl.[3]: **C 07 D 271/08**, A 61 K 31/42

(21) Anmeldenummer: **81110399.3**

(22) Anmeldetag: **12.12.81**

(54) **3,4-Bis-substituierte 1,2,5-Oxadiazol-2-oxide, Verfahren zu ihrer Herstellung, und sie enthaltende pharmazeutische Zubereitungen.**

(30) Priorität: **18.12.80 DE 3047730**

(43) Veröffentlichungstag der Anmeldung: **30.06.82 Patentblatt 82/26**

(45) Bekanntmachung des Hinweises auf die Patenterteilung: **31.10.84 Patentblatt 84/44**

(84) Benannte Vertragsstaaten: **AT BE CH DE FR GB IT LI NL SE**

(56) Entgegenhaltungen: **US - A - 4 089 867**

(73) Patentinhaber: **CASSELLA Aktiengesellschaft, Hanauer Landstrasse 526, D-6000 Frankfurt am Main 61 (DE)**

(72) Erfinder: **Schönafinger, Karl, Dr., Parkstrasse 1, D-8531 Uehlfeld (DE)**
Erfinder: **Beyerle, Rudi, Dr., An der Pfaffenmauer 44, D-6000 Frankfurt am Main 60 (DE)**
Erfinder: **Mogilev, Anton, Dr., Diaminstrasse 6, D-6000 Frankfurt am Main 61 (DE)**
Erfinder: **Bohn, Helmut, Dr., Kranzbergring 11, D-6369 Schöneck (DE)**
Erfinder: **Just, Melitta, Dr., Hüttenbergweg 6, D-6369 Schöneck 1 (DE)**
Erfinder: **Martorana, Piero A., Dr., Kaiser-Friedrich-Promenade 108, D-6380 Bad Homburg (DE)**
Erfinder: **Nitz, Rolf-Eberhard, Dr., Heinrich-Bingemer-Weg 64, D-6000 Frankfurt am Main 60 (DE)**

(74) Vertreter: **Urbach, Hans-Georg, Dr. et al, Hanauer Landstrasse 526, D-6000 Frankfurt am Main 61 (DE)**

**EP 0 054 872 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft 3,4-bis-substituierte 1,2,5-Oxadiazol-2-oxide der Formel I

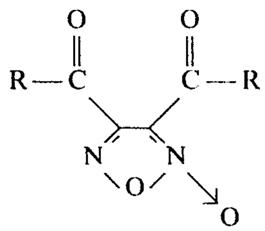

(I)

und ihre pharmakologisch annehmbaren Säureadditionsverbindungen, Verfahren zu ihrer Herstellung, und sie erhaltende pharmazeutische Zubereitungen.

In der Formel I bedeutet

R = $-NHR^1$, $-NR^2R^3$, $-NHR^4OR^2$, $-NHR^5COR^6$,

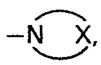

$R^1$ = Alkyl mit 1 bis 6 C-Atomen, Cycloalkyl mit 4 bis 7 C-Atomen,
$R^2$, $R^3$ = Alkyl mit 1 bis 4 C-Atomen
$R^4$ = einen Alkylenrest der Formel $-C_nH_{2n}-$, wobei n 2, 3 oder 4 bedeutet,
$R^5$ = einen Alkylenrest der Formel $-C_mH_{2m}-$, wobei m 1, 2 oder 3 bedeutet,
$R^6$ = $-OR^2$, $-NHR^1$, $-NH_2$, $-NR^2R^3$,
X = $-(CH_2)_p-$, $-(CH_2)_2-O-(CH_2)_2-$,

$$-(CH_2)_2-\underset{|}{\overset{R^2}{N}}-(CH_2)_2-$$

p = 4, 5 oder 6.

Die für $R^1$, $R^2$, $R^3$ stehenden Alkylreste sowie die für $R^4$ stehenden Alkylenreste können geradkettig oder verzweigt sein. Von den Verbindungen der Formel I sind diejenigen bevorzugt, die in den R-Resten eine —NH-Gruppierung enthalten. Vorzugsweise steht $R^4$ für $-(CH_2)_2-$ oder $-(CH_2)_3-$ und $R^5$ für $-CH_2-$ oder $-(CH_2)_2-$. $R^2$ bedeutet vorzugsweise Methyl oder Ethyl.

Die erfindungsgemäßen Verbindungen der Formel I lassen sich aus Hydroxamoylchloriden der Formel II durch HCl-Abspaltung und anschließend Dimerisierung herstellen:

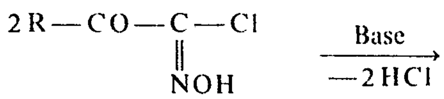


(II) (I)

Die Reaktion wird in einem geeigneten Lösungs- oder Dispergiermittel, wie z. B. Dimethylformamid, Dimethylsulfoxid, N-Methylpyrrolidon, niederen Alkohol wie Methanol oder Ethanol, vorzugsweise aber in Wasser durchgeführt. Auch Mischungen verschiedener Lösungs- oder Dispergiermittel, insbesondere homogene aber auch heterogene Mischungen mit Wasser, wie z. B. Wasser/Methanol oder Wasser/Diethyläther, können verwendet werden. Die Reaktion wird im allgemeinen bei 0 bis +50°C, vorzugsweise bei 10 bis 25°C, durchgeführt.

Nach dem Eintragen der Verbindung II ist das Lösungs- oder Dispergiermittel läuft die Reaktion häufig von selbst ab. Sie kann jedoch durch Zusatz einer Base, die den abgespaltenen Chlorwasserstoff bindet, erheblich beschleunigt werden. Als derartige Base können z. B. benutzt werden: sekundäre oder tertiäre organische Amine, wie z. B. Dimethylamin, Trimethylamin, Diethylamin, Triethylamin, Pyridin; Alkalihydroxyde, wie z. B. Natrium- oder Kaliumhydroxyd; Alkalikarbonate und Alkalibikarbonate, wie z. B. Pottasche, Soda und Natriumbikarnonat; Alkaliacetate, wie z. B. Natriumacetat. Soda und Natriumbikarbonat sind bevorzugt. Die Base kann auch in Form einer Lösung (z. B. einer wäßrigen Lösung wie im Falle der niederen organischen Amine) oder einer Dispersion der Lösung oder Dispersion der Verbindung II zugefügt werden. Wegen der eintretenden Reaktion ist eine allmähliche oder portionsweise Zugabe der Base und Umrühren des Reaktions-Gemisches zweckmäßig. Die Base wird vorzugsweise in molarer Menge (auf 2 Mole der Verbindung II 2 Mole Base), gegebenenfalls mit einem bis zum 20%igen Überschuß zugesetzt. Nach beendeter Reaktion wird die gebildete Verbindung der Formel I abgetrennt und gewünschtenfalls in eine Säureadditionsverbindung überführt.

Die benötigten Hydroxamoyl-chlorid-Ausgangsverbindungen der Formel II können nach verschiedenen an sich bekannten Verfahren hergestellt werden, beispielsweise dadurch, daß zunächst Diketten III mit einem Amin IV zu einem Acetessigamid V umgesetzt wird (vgl. z. B. US-PS 2 174 239):

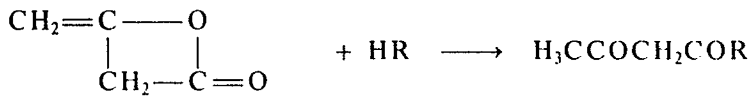

(III) (IV) (V)

Die Umsetzung zwischen den Verbindungen III und IV wird in einem geeigneten Lösungsmittel, wie z. B. Wasser oder einem Alkohol, normalerweise bei Temperaturen von 10 bis 50°C, vorzugsweise bei Raumtemperaturen durchgeführt.

Das Acetessigamid V wird durch in situ erzeugte salpetrige Säure in einem geeigneten Lösungsmittel wie Wasser, Eisessig oder einem Alkohol oximiert, wobei Oxim VI entsteht:

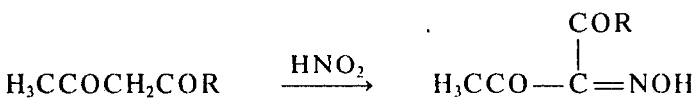


(V) (VI)

Diese Reaktion wird z. B. bei einem pH-Wert von nicht weniger als 4,0 und bei Temperaturen von 10 bis 50°C, vorzugsweise bei Raumtemperatur, durchgeführt.

Die salpetrige Säure wird am einfachsten aus Natrium-nitrit und Salzsäure erzeugt.

Das Oxim VI wird nun in einem geeigneten Lösungsmittel, wie Wasser oder einem Alkohol, z. B. bei Temperaturen von +10 bis 70°C, vorzugsweise 30 bis 50°C, chloriert, wobei das Hydroxamoyl-chlorid II entsteht.

Das Hydroxamoyl-chlorid II kann aus dem Acetessigamid V auch so hergestellt werden, daß man die Reihenfolge der Oximierung und Chlorierung vertauscht. Bei der Chlorierung entsteht dann zunächst aus dem Acetessigamid V die Chlorverbindung VII: $CH_3COCHClCOR$, die dann durch Umsetzung mit salpetriger Säure in die Verbindung II überführt wird.

Die Überführung von Acetessigamiden V in Verbindungen der Formel II nach den oben angegebenen Möglichkeiten ist z. B. beschrieben in der Deutschen Auslegeschrift 1 963 061. Ausgehend von Diketten III und dem Amin IV können die erfindungsgemäßen Verbindungen nach den vorstehend angegebenen Syntheseschritten ohne Isolierung der Zwischenprodukte in einem einzigen Reaktionsgefäß durchgeführt werden. Die Ausbeuten über alle Stufen gerechnet liegen im allgemeinen bei 20 bis 70 % d. Th.

Die Hydroxamoylchloride II können auch durch Umsetzung eines Glyoxylsäureesteroxims VII mit einem Amin IV hergestellt werden, wobei zunächst die

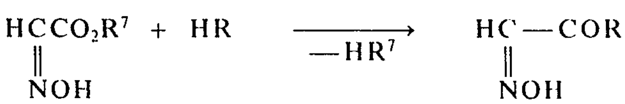


(VII) (IV) (VIII)

Oximverbindung VIII entsteht. $R^7$ bedeutet niederes Alkyl, insbesondere Methyl oder Ethyl. Die Verbindung VIII wird dann durch Chlorierung in die Verbindung II überführt. Die Erfindungsgemäßen Verbindungen I, die eine basische Seitenkette aufweisen, bilden mit anorganischen oder organischen Säuren Salze. Solche Säuren sind beispielsweise: Chlorwasserstoff-, Bromwasserstoff-, Phosphor-, Schwefel-, Oxal-, Milch-, Wein-, Essig-, Salicyl-, Benzoe-, Zitronen-, Ascorbin-, Adipin- und Naphthalindisulfonsäure. Die Säureadditionsverbindungen werden in bekannter Weise durch Vereinigen der Komponenten in einem geeigneten Lösungs- oder Dispergiermittel erhalten.

Die Verbindungen der Formel I und ihre pharmakologisch annehmbaren Säureadditionsverbindungen besitzen wertvolle pharmakologische Eigenschaften. Besonders ausgeprägt ist ihre Wirkung auf das Herzkreislaufsystem. Sie senken in niedriger Dosierung den Blutdruck, vermindern den peripheren Widerstand und führen über eine Senkung des Pulmonalarteriendrucks bei nur wenig beeinflußter Herzfrequenz zu einer Verminderung der Herzarbeit.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z. B. in Form von Pillen, Tabletten, Lacktabletten, Dragees, Hart- und Weichgelantinekapseln, Lösungen, Sirup, Emulsionen oder Suspensionen, oder als Aerosolmischungen verabreicht werden. Die Verabreichung kann aber auch rektal, z. B. in Form von Suppositorien, oder parenteral, z. B. in Form von Injektionslösungen, oder perkutan, z. B. in Form von Salben oder Tinkturen, erfolgen.

Zur Herstellung der pharmazeutischen Präparate werden pharmazeutisch inerte anorganische oder organische Trägerstoffe verwendet. Für die Herstellung von Pillen, Tabletten, Dragees und Hartgelantinekapseln verwendet man z. B. Lactose, Maisstärke oder Derivate, davon Talk, Stearinsäure oder deren Salze etc. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich z. B. Wasser, Alkohole, Glycerin, Polyole, pflanzliche Öle etc.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z. B. Propranolol, Pindolol, Metoprolol; Vasodilatatoren, wie z. B. Carbochromen; Beruhigungsmittel, wie z. B. Barbitursäurederivate, 1,4-Benzodiazepine und Meprobamat; Diuretica, wie z. B. Chlorothiazid; das Herz tonisierene Mittel, wie z. B. Digitalispräparate; blutdrucksenkende Mittel, wie z. B. Hydralazin, Dihydralazin, Prazosin, Clonidin, Rauwolfia-Alkaloide; Mittel, die den Fettsäurespiegel im Blut senken, wie z. B. Bezafibrat, Fenofibrat; Mittel für die Thromboseprophylaxe, wie z. B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,2 bis 150 mg, vorzugsweise 1 bis 30 mg, angemessen. Auch bei anderen Applikationsformen liegt die Tagesdosis, wegen der guten Resorption der Wirkstoffe, in ähnlichen Mengenbereichen, d. h. im allgemeinen ebenfalls bei 0,2 bis 150 mg/Mensch. Die Tagesdosis wird normalerweise in mehrere, z. B. 2 oder 3 Teilverabreichungen aufgeteilt.

Die pharmazeutischen Zubereitungen enthalten von den Wirkstoffen der Formel I oder ihren pharmakologisch annehmbaren Säureadditionssalzen im allgemeinen 0,1 bis 50 mg, vorzugsweise 0,5 bis 10 mg/Dosis.

Die Untersuchungen über die antianginöse und antihypertensive Wirkung der Verbindungen der Formel I wurden an Bastardhunden beiderlei Geschlechts in Pentobarbital-Narkose (30 bis 40 mg/kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit dem Uras) betrug zwischen 4,5 und 5 Vol.-%. Während des gesamten Versuchs erhielten die Tiere mit Pentobarbital-Narkose eine Dauerinfusion von Pentobarbital i.v. = 4 mg/kg/6 ml/h, um eine konstante Narkosetiefe zu gewährleisten; die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Der systolische und diastolische Blutdruck wurde peripher in der Arteria femoralis über einen Statham-Druckaufnehmer gemessen. Ein über die Arteria carotis in den linken Ventrikel geschobener Millar-Tip-Katheter lieferte das Signal für den LVEDP (linksventrikulärer enddiastolischer Druck) und die Herzfrequenz. Mit einem zweiten, über die Vena jugularis eingeschobenen Tip-Katheter wurde der mittlere Blutdruck in der Arteria pulmonalis erfaßt. Die erhaltenen Ergebnisse sind in der folgenden Tabelle angegeben.

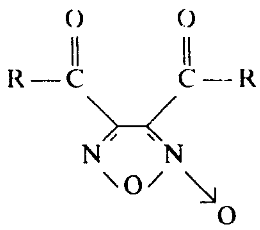

| R | Dosis mg/kg | LVEDP ΔmmHg | PAP ΔmmHg | BDm ΔmmHg | HF Δb/min | t/min |
|---|---|---|---|---|---|---|
| 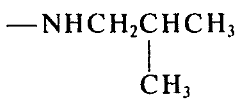 | 0,05 | −2 | −3 | −70 | – | 60 |
| —NH—Cyclohexyl | 0,05 | −3,5 | −3 | −65 | − 5 | 45 |
| 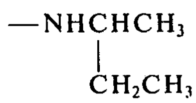 | 0,05 | −2 | −3 | −75 | −10 | 75 |
| 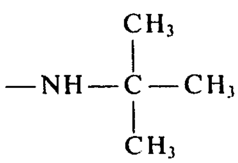 | 0,05 | −2 | −2 | −60 | + 5 | 60 |
| $—NHCH_2CO_2CH_3$ | 0,05 | −5 | −3 | −65 | +10 | 45 |
| $—NHCH_2CH_3$ | 0,05 | −7 | −3 | −85 | − 5 | 60 |
| Piperidino | 0,05 | – | −1 | −25 | −20 | >120 |
| $—NHC_4H_{9(n)}$ | 0,05 | −5 | −2,5 | −70 | −10 | 30 |
| 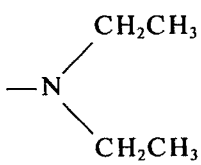 | 0,1 | −2,5 | −1 | −25 | − 5 | 35 |
| $—NHCH_2CH_2OCH_3$ | 0,05 | −4 | −1,5 | −65 | +30 | 40 |
| 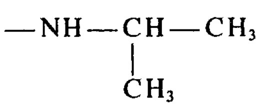 | 0,05 | −2,3 | −1,5 | −45 | − 1 | 75 |
| $—NHCH_3$ | 0,05 | −7 | −4 | −55 | −15 | 60 |
| ISDN | 0,05 | −2,1 | −0,7 | −19 | ± 0 | 30 |

In der Tabelle bedeuten:

LVEDP = linksventrikulärer enddiastolischer Druck
PAP = mittlerer Pulmonalarteriendruck
BDm = mittlerer peripherer Blutdruck
HF = Herzfrequenz
t/min = mittlere Wirkdauer
ISDN = Isosorbiddinitrat (Vergleichssubstanz)

Das folgende Beispiel dient der weiteren Erläuterung der Erfindung.

Beispiel

1,2,5-Oxadiazol-2-oxid-3,4-bis-(carbonsäure-methylamid). 12,5 g Methylamin werden in 400 ml Wasser gelöst. 34 g Diketen werden bei Raumtemperatur (20 °C), langsam zugetropft, wobei sich ein stabiler pH-Wert von 7 einstellt. In der erhaltenen Lösung werden dann 28 g Natriumnitrit gelöst. Konzentrierte Salzsäure (40 g) wird dann so zugetropft, daß der pH-Wert der Lösung nicht unter 4 absinkt. Nun wird 30 Min. lang nachgerührt und anschließend bei 20—40 °C 30 g Chlor eingeleitet. Nach Abkühlen der Lösung wird auf —10 °C abgekühlt und der Feststoff abgesaugt. Er wird in 100 ml Wasser aufgeschlämmt und bei 20 °C vorsichtig, portionsweise mit insgesamt 33,6 g Natriumbicarbonat versetzt. Die Lösung besitzt nun einen pH-Wert von 7,5—8. Nun wird auf 0 °C abgekühlt und der ausgefallene Feststoff abgesaugt und aus Isopropanol umkristallisiert. Farblose Kristalle vom Fp. 164—165 °C.

In analoger Weise können die in der nachfolgenden Tabelle angegebenen Verbindungen bei den genannten Reaktionstemperaturen in den angegebenen Lösungsmitteln und unter Verwendung der genannten Basen hergestellt werden.

Die Strukturen der synthetisierten Verbindungen können durch Elementaranalyse und durch die IR- und NMR-Spektren bestätigt werden.

| 1,2,5-Oxadiazol-2-oxid-3,4-bis-carbonsäureamid | Schmelzpunkt in °C | Reaktionstemperatur in °C | Lösungsmittel | Base |
|---|---|---|---|---|
| Bis-dimethylamid | 127—129 | 30 | $H_2O$ | $NaHCO_3$ |
| Bis-diethylamid | Öl | 35 | $H_2O$ | $NaHCO_3$ |
| Bis-morpholid | 127—139 | 30 | $H_2O$ | $NaHCO_3$ |
| Bis-butylamid | 91—92 | 25 | $H_2O/C_2H_5OC_2H_5$ | $NaHCO_3$ |
| Bis-pyrrolidid | 106—108 | 30 | $(CH_3)_2SO$ | $Na_2CO_3$ |
| Bis-ethylamid | 116—117 | 0 | DMF | $KHCO_3$ |
| Bis-isopropylamid | 128—131 | 20 | $H_2O$ | $(i\text{-}C_3H_7)_2NH$ |
| Bis-(2-methoxyethyl)amid | 94—95 | 10 | $C_2H_5OH$ | $CH_3COONa$ |
| Bis-piperidid | 115—116 | 25 | $H_2O$ | $CH_3COOK$ |
| Bis-tert.-butylamid | 166—167 | 50 | $CH_3OH$ | $(CH_3)_3N$ |
| Bis-cyclohexylamid | 137—139 | 45 | $H_2O/C_2H_5OC_2H_5$ | NaOH |
| Bis-isobutylamid | 83,5—87 | 10 | NMP | $K_2CO_3$ |
| Bis-sek-butylamid | Öl | 10 | NMP | $NaHCO_3$ |
| Bis-(methoxycarbonyl-methyl)amid | 97—99 | 20 | $CH_3OH$ | KOH |
| Bis-cyclopentylamid | 114—146 | 30 | $H_2O/C_2H_2H_5OH$ | $NaHCO_3$ |
| Bis-propylamid | 117—118 | 20 | $H_2O$ | $NaHCO_3$ |
| Bis-(4-methyl)piperazid | 240 (Zers) Dihydrochlorid | 25 | $C_2H_5OH$ | $NaHCO_3$ |
| Bis-(3-methoxypropyl)amid | 65—68 | 10 | $H_2O$ | $NaHCO_3$ |
| Bis-(4-methoxybutyl)amid | Öl | 20 | $H_2O$ | $NaHCO_3$ |
| Bis-hexamethylenimid | 103—106 | 25 | $H_2O/C_2H_5OC_2H_5$ | $NaHCO_3$ |

Fortsetzung

| 1,2,5-Oxadiazol-2-oxid-3,4-bis-carbonsäureamid | Schmelzpunkt in °C | Reaktionstemperatur in °C | Lösungsmittel | Base |
|---|---|---|---|---|
| Bis-(2-methoxycarbonylethyl)amid | 80–82 | 10 | $CH_3OH$ | $NaHCO_3$ |
| Bis-(2-methoxycarbonylpropyl)amid | Öl | 20 | $CH_3OH$ | $K_2CO_3$ |
| Bis-(diethylaminocarbonylmethyl)amid | Öl | 25 | n-$C_3H_7OH$ | $K_2CO_3$ |
| Bis-(aminocarbonylmethyl)-amid | 141–142 | 20 | $CH_2OH$ | $NaHCO_3$ |
| Bis-(di-n-butyl)amid | Öl | 30 | $H_2O/C_2H_5OC_2H_5$ | $NaHCO_3$ |
| Bis-(ethylaminocarbonyl)-methyl)amid | Öl | 25 | $H_2O$ | $NaHCO_3$ |
| Bis-(2-ethoxyethyl)amid | Öl | 20 | $H_2O/C_2H_5OC_2H_5$ | $NaHCO_3$ |
| Bis-(2-ethoxycarbonylethyl)-amid | Öl | 20 | $C_2H_5OH$ | $K_2CO_3$ |

DMF = Dimethylformamid
NMP = N-Methylpyrrolidon

**Patentansprüche für die Vertragsstaaten: BE, CH, DE, FR, GB, IT, LI, NL, SE**

1. 3,4-bis-substituierte 1,2,5-Oxadiazol-2-oxide der Formel

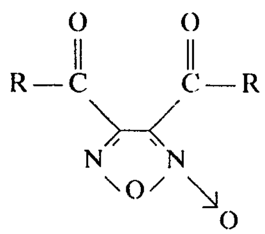

(I)

und ihre pharmakologisch annehmbaren Säureadditionsverbindungen, worin

R = $-NHR^1$, $-NR^2R^3$, $-NHR^4OR^2$, $-NHR^5COR^6$,

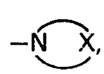

$R^1$ = Alkyl mit 1 bis 6 C-Atomen, Cycloalkyl mit 4 bis 7 C-Atomen,
$R^2$, $R^3$ = Alkyl mit 1 bis 4 C-Atomen,
$R^4$ = einen Alkylenrest der Formel $-C_nH_{2n}-$, wobei n 2, 3 oder 4 bedeutet,
$R^5$ = einen Alkylenrest der Formel $-C_mH_{2m}-$, wobei m 1, 2 oder 3 bedeutet,
$R^6$ = $-OR^2$, $-NHR^1$, $-NR^2R^3$, $-NH_2$,
X = $-(CH_2)_p-$, $-(CH_2)_2-O-(CH_2)_2-$,

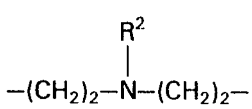

p = 4, 5 oder 6, bedeuten.

2. 3,4-Bis-substituierte 1,2,5-Oxadiazol-2-oxide nach Anspruch 1, dadurch gekennzeichnet, daß die Reste R eine $-NH$-Gruppierung enthalten.

3. 3,4-Bis-substituierte 1,2,5-Oxadiazol-2-oxide nach den Ansprüchen 1 und/oder 2, dadurch gekennzeichnet, daß $R^4$ $-(CH_2)_2-$ oder $-(CH_2)_3-$ bedeutet.

4. 3,4-Bis-substituierte 1,2,5-Oxadiazol-2-oxide nach den Ansprüchen 1 und/oder 2, dadurch gekennzeichnet, daß $R^5$ $-CH_2-$ oder $-(CH_2)_2-$ bedeutet.

5. 1,2,5-Oxadiazol-2-oxid-3,4-bis-(carbonsäure-isopropylamid).

6. Verfahren zur Herstellung der 3,4-bis-substituierten 1,2,5-Oxadiazol-2-oxide nach einem oder mehreren der Ansprüche 1 bis 5, durch Chlorwasserstoffabspaltung aus einem Hydroxamoylchlorid der Formel II

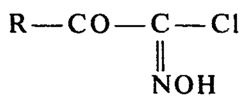

(II)

und Dimerisierung, sowie gegebenenfalls anschließende Überführung in die Säureadditionsverbindung.

7. Verfahren nach Anspruch 6, dadurch gekennzeichnet, daß die Reaktion in einem Lösungs- oder Dispergiermittel bei Temperaturen von 0 bis 50°C, vorzugsweise von 10 bis 25°C, durchgeführt wird.

8. Verfahren nach den Ansprüchen 6 und/oder 7, dadurch gekennzeichnet, daß bei der Reaktion eine Base, vorzugsweise in molarer Menge, zugesetzt wird.

9. 3,4-Bis-substituierte 1,2,5-Oxadiazol-2-oxide nach einem oder mehreren der Ansprüche 1 bis 5 oder ihre pharmakologisch annehmbaren Säureadditionsverbindungen zur Verwendung als pharmakologische Wirkstoffe.

10. Pharmazeutische Zubereitung, enthaltend eine wirksame Dosis eines 3,4-bis-substituierten 1,2,5-Oxadiazol-2-oxids nach einem oder mehreren der Ansprüche 1 bis 5, oder einer Säureadditionsverbindung davon, neben pharmazeutisch zulässigen Träger- und Zusatzstoffen und gegebenenfalls noch anderen therapeutisch wirksamen Substanzen.

**Patenansprüche für den Vertragsstaat: AT**

1. Verfahren zur Herstellung von 3,4-bis-substituierten 1,2,5-Oxadiazol-2-oxiden der Formel I

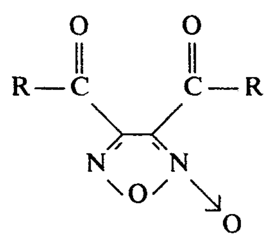

(I)

und ihrer pharmakologisch annehmbaren Säureadditionsverbindungen, worin

R = $-NHR^1$, $-NR^2R^3$, $-NHR^4OR^2$, $-NHR^5COR^6$,

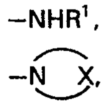,

$R^1$ = Alkyl mit 1 bis 6 C-Atomen, Cycloalkyl mit 4 bis 7 C-Atomen,

$R^2$, $R^3$ = Alkyl mit 1 bis 4 C-Atomen,

$R^4$ = einen Alkylenrest der Formel $-C_nH_{2n}-$, wobei n 2, 3 oder 4 bedeutet,

$R^5$ = einen Alkylenrest der Formel $-C_mH_{2m}-$, wobei m 1, 2 oder 3 bedeutet,

$R^6$ = $-OR^2$, $-NHR^1$, $-NR^2R^3$, $-NH_2$,

X = $-(CH_2)_p-$, $-(CH_2)_2-O-(CH_2)_2-$,

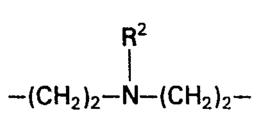

p = 4, 5 oder 6, bedeuten,

dadurch gekennzeichnet, daß ein Hydroxamoylchlorid der Formel II

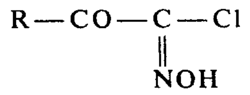

(II)

in ein Lösungs- oder Dispergiermittel bei Temperaturen von 0 bis 50°C eingetragen wird, wodurch unter HCl Abspaltung und Dimerisierung eine Verbindung der Formel I gebildet wird, die gegebenenfalls in an sich bekannter Weise in ein Säureadditionssalz überführt wird.

2. Verfahren nach Anspruch 1, dadurch gekennzeichnet, daß das Hydroxamoylchlorid der Formel II in das Lösungs- oder Dispergiermittel bei Temperaturen von 10 bis 25°C eingetragen wird.

3. Verfahren nach den Ansprüchen 1 und/oder 2, dadurch gekennzeichnet, daß eine Base, vorzugsweise in molarer Menge, zugesetzt wird.

4. Verfahren nach Anspruch 3, dadurch gekennzeichnet, daß als Base ein sekundäres oder tertiäres Amin, Alkalihydroxid, Alkalicarbonat, Alkalibicarbonat oder Alkaliacetat zugefügt wird.

5. Verfahren nach Anspruch 3 und/oder 4, dadurch gekennzeichnet, daß die Base allmählich oder portionsweise zugefügt wird.

6. Verfahren nach einem der Ansprüche 1 bis 5, dadurch gekennzeichnet, daß als Lösungsmittel Dimethylformamid, Dimethylsulfoxid, N-Methylpyrrolidon, ein niederer Alkohol, Diethylether, Wasser oder ein Gemisch dieser Lösungsmittel verwendet wird.

7. Verfahren nach einem oder mehreren der Ansprüche 1 bis 6, dadurch gekennzeichnet, daß der Rest R eine —NH-Gruppierung enthält.

8. Verfahren nach einem oder mehreren der Ansprüche 1 bis 7, dadurch gekennzeichnet, daß $R^4$ $-(CH_2)_2-$ oder $-(CH_2)_3-$ bedeutet.

9. Verfahren nach einem oder mehreren der Ansprüche 1 bis 7, dadurch gekennzeichnet, daß $R^5$ $-CH_2-$ oder $-(CH_2)_2-$ bedeutet.

10. Verfahren nach einem oder mehreren der Ansprüche 1 bis 7, dadurch gekennzeichnet, daß $R^2$ Methyl oder Ethyl bedeutet.

**Patent Claims for Contracting States: BE, CH, DE, FR, GB, IT, LI, NL, SE**

1. 3,4-Bis-substituted 1,2,5-oxadiazole 2-oxides of the formula

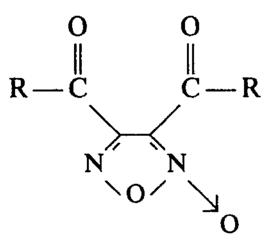

(I)

and their pharmacologically acceptable acid addition compounds wherein

R denotes $-NHR^1$, $-NR^2R^3$, $-NHR^4OR^2$, $-NHR^5COR^6$ or

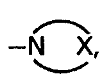

$R^1$ denotes alkyl having 1 to 6 C atoms or cycloalkyl having 4 to 7 C atoms,
$R^2$ and $R^3$ denote alkyl having 1 to 4 C atoms,
$R^4$ denotes an alkylene radical of the formula $-C_nH_{2n}-$ wherein n denotes 2, 3 or 4,
$R^5$ denotes an alkylene radical of the formula $-C_mH_{2m}-$ wherein m denotes 1, 2 or 3,
$R^6$ denotes $-OR^2$, $-NHR^1$, $-NR^2R^3$ or $-NH_2$,
X denotes $-(CH_2)_p-$, $-(CH_2)_2-O-(CH_2)_2-$ or

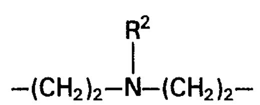

and

p is 4, 5 or 6.

2. 3,4-Bis-substituted 1,2,5-oxadiazole 2-oxides according to Claim 1, characterised in that the radicals r contain an —NH-grouping.

3. 3,4-Bis-substituted 1,2,5-oxadiazole 2-oxides according to Claims 1 and/or 2, characterised in that $R^4$ denotes $-(CH_2)_2-$ or $-(CH_2)_3-$.

4. 3,4-Bis-substituted 1,2,5-oxadiazole 2-oxides according to Claims 1 and/or 2, characterised in that $R^5$ denotes $-CH_2-$ or $-(CH_2)_2-$.

5. 1,2,5-Oxadiazole 2-oxide-3,4-bis-(carboxylic acid isopropylamide).

6. Process for the preparation of the 3,4-bis-substituted 1,2,5-oxadiazole 2-oxides of one or several of Claims 1 to 5, by eliminating hydrogen chloride from a hydroxamoyl chloride of the formula II

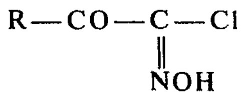

(II)

and dimerisation and also, if desired, subsequent conversion into the acid addition compound.

7. Process according to Claim 6, characterised in that the reaction is carried out in a solvent or dispersing agent at temperatures from 0 to 50°C, preferably from 10 to 25°C.

8. Process according to Claims 6 and/or 7, characterised in that a base, preferably in a molar quantity, is added for the reaction.

9. 3,4-Bis-substituted 1,2,5-oxadiazole 2-oxides according to one or several of Claims 1 to 5, or their pharmacologically acceptable acid addition compounds for use as pharmacological active compoumds.

10. Pharmaceutical formulation containing an active dose of a 3,4-bis substituted 1,2,5-oxadiazole 2-oxide according to one or several of Claims 1 to 5, or of an acid addition compound thereof, together with pharmaceutically permissible excipients and additives and also, if desired, other therapeutically active substances.

**Patent Claims for the Contracting State: AT**

1. Process for the preparation of 3,4-bis-substituted 1,2,5-oxidiazole 2-oxides of the formula

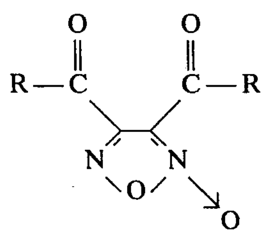

(I)

and their pharmacologically acceptable acid addition compounds wherein

R denotes $-NHR^1$, $-NR^2R^3$, $-NHR^4OR^2$, $-NHR^5COR^6$ or

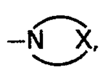

$R^1$ denotes alkyl having 1 to 6 C atoms or cycloalkyl having 4 to 7 C atoms,
$R^2$ and $R^3$ denote alkyl having 1 to 4 C atoms,
$R^4$ denotes an alkylene radical of the formula $-C_nH_{2n}-$ wherein n denotes 2, 3 or 4,
$R^5$ denotes an alkylene radical of the formula $-C_mH_{2m}-$ wherein m denotes 1, 2 or 3,
$R^6$ denotes $-OR^2$, $-NHR^1$, $-NR^2R^3$ or $-NH_2$,
X denotes $-(CH_2)_p-$, $-(CH_2)_2-O-(CH_2)_2-$ or

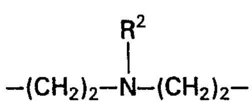

and

p is 4, 5 or 6,

characterised in that a hydroxamoyl chloride of the formula II

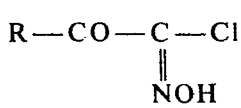

(II)

is introduced into a solvent or dispersing agent at temperatures from 0 to 50°C whereupon a compound

of the formula I is formed with elimination of HCl and dimerisation, which compound is, if desired, converted in a manner known per se into an acid addition salt.

2. Process according to Claim 1, characterised in that the hydroxamoyl chloride of formula II is introduced into the solvent or dispersing agent at temperatures from 10 to 25°C.

3. Process according to Claims 1 and/or 2, characterised in that a base, preferably in a molar quantity, is added.

4. Process according to Claim 3, characterised in that the base added is secondary or tertiary amine, alkali metal hydroxide, alkali metal carbonate, alkali metal bicarbonate or alkali metal acetate.

5. Process according to Claims 3 and/or 4, characterised in that the base is added gradually or in portions.

6. Process according to one of Claims 1 to 5, characterised in that the solvent used is dimethylformamide, dimethylsulphoxide, N-methylpyrrolidone, a lower alcohol, diethylether, water or a mixture of these solvents.

7. Process according to one or several of Claims 1 to 6, characterised in that the radical R contains an —NH-grouping.

8. Process according to one or serveral of Claims 1 to 7, characterised in that $R^4$ denotes $-(CH_2)_2-$ or $-(CH_2)_3-$.

9. Process according to one or several of Claims 1 to 7, characterised in that $R^5$ denotes $-CH_2-$ or $-(CH_2)_2-$.

10. Process according to one or several of Claims 1 to 7, characterised in that $R^2$ denotes methyl or ethyl.

**Revendications pour les Etats contractants: BE, CH, DE, FR, GB, IT, LI, NL, SE**

1. Oxadiazole-1,2,5 oxydes-2 disubstitués en 3,4 qui répondent à la formule:

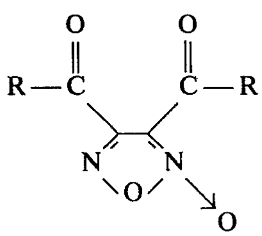

(I)

dans laquelle:

R représente $-NHR^1$, $-NR^2R^3$, $-NHR^4OR^2$, $-NHR^5COR^6$ ou

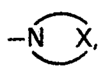

$R^1$ représente un alkyle contenant de 1 à 6 atomes de carbone ou un cycloalkyle contenant de 4 à 7 atomes de carbone,

$R^2$ et $R^3$ représentent chacun un alkyle contenant de 1 à 4 atomes de carbone,

$R^4$ représente un radical alkylène $-C_nH_{2n}-$ dans lequel n désigne un nombre égal à 2, à 3 ou à 4,

$R^5$ représente un radical alkylène $-C_mH_{2m}-$ dans lequel m désigne un nombre égal 1, à 2 ou à 3,

$R^6$ représente $-OR^2$, $-NHR^1$, $-NR^2R^3$ ou $-NH_2$,

X représente $-(CH_2)_p-$, $-(CH_2)_2-O-(CH_2)_2-$ ou

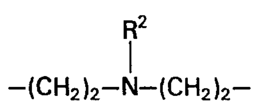

et

p représente un nombre égal à 4, à 5 ou à 6, ainsi

que leurs composés d'addition d'acides acceptables du point de vue pharmacologique.

2. Oxadiazole-1,2,5 oxydes-2 disubstitués en 3,4 selon la revendication 1, caractérisés en ce que les radicaux R contiennent un radical —NH—.

3. Oxadiazole-1,2,5 oxydes-2 disubstitués en 3,4 selon l'une des revendications 1 et/ou 2, caractérisés en ce que $R^4$ représente un radical $-(CH_2)_2-$ ou $-(CH_2)_3-$.

4. Oxadiazole-1,2,5 oxydes-2 disubstitués en 3,4 selon l'une des revendications 1 et/ou 2, caractérisés en ce que $R^5$ représente un radical $-CH_2-$ ou $-(CH_2)_2-$.

5. Bis-(isopropylaminocarbonyl)-3,4 oxidiazole-1,2,5 oxyde-2.

6. Procédé de préparation des oxadiazole-1,2,5 oxydes-2 disubstitués en 3,4 selon l'une quelconque des revendications 1 à 5, par déshydrochloration à partir d'un chlorure d'hydroxamoyle de formule (II):

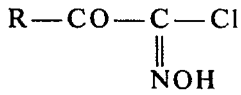

(II)

et dimérisation, puis transformation éventuelle en composé d'addition d'acide.

7. Procédé selon la revendication 6, caractérisé en ce qu'on effectue la réaction dans un solvant ou milieu de dispersion à des températures de 0 à 50°C, de préférence de 10 à 25°C.

8. Procédé selon l'une des revendications 6 et/ou 7, caractérisé en ce qu'on ajoute, lors de la réaction, une base, de préférence en la quantité molaire.

9. Oxadiazole-1,2,5 oxydes-2 disubstitués en 3,4 selon l'une quelconque des revendications 1 à 5, ou leurs composés d'addition d'acides acceptables du point de vue pharmacologique, pour l'application comme substances actives pharmacologiques.

10. Compositions pharmaceutiques contenant une dose efficace d'un oxadiazole-1,2,5 oxyde-2 disubstitué en 3,4 selon l'une quelconque des revendications 1 à 5, ou d'un composé d'addition d'acide d'un tel oxadiazole, ainsi que des excipients et additifs acceptables du point de vue pharmaceutique et, éventuellement, d'autres substances douées d'activités thérapeutiques.

**Revendications pour l'Etat contractant: AT**

1. Procédé de préparation d'oxadiazole-1,2,5 oxydes-2 disubstitués en 3,4 qui répondent à la formule (I):

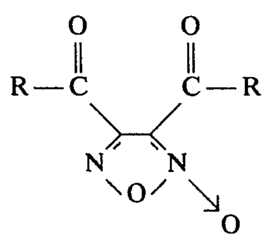

(I)

dans laquelle:

R représente $-NHR^1$, $-NR^2R^3$, $-NHR^4OR^2$, $-NHR^5COR^6$ ou

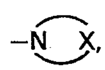

$R^1$ représente un alkyle contenant de 1 à 6 atomes de carbone ou un cycloalkyle contenant de 4 à 7 atomes de carbone,

$R^2$ et $R^3$ représentent chacun un alkyle contenant de 1 à 4 atomes de carbone,

$R^4$ représente un radical alkylène $-C_nH_{2n}-$ dans lequel n désigne un nombre égal à 2, à 3 ou à 4,

$R^5$ représente un radical alkylène $-C_mH_{2m}-$ dans lequel m désigne un nombre égal 1, à 2 ou à 3,

$R^6$ représente $-OR^2$, $-NHR^1$, $-NR^2R^3$ ou $-NH_2$,

X représente $-(CH_2)_p-$, $-(CH_2)_2-O-(CH_2)_2-$ ou

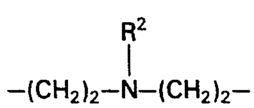 et

p représente un nombre égal à 4, à 5 ou à 6,

et de leurs composés d'addition d'acides acceptables du point de vue pharmacologique, procédé caractérisé en ce qu'on introduit un chlorure d'hydroxamoyle der formule (II):

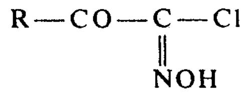

(II)

dans un solvant ou milieu de dispersion, à des températures de 0 à 50°C, opération qui a pour effet de donner naissance, avec enlèvement d'HCl et dimérisation, à un composé de formule (I), que l'on transforme éventuellement, de manière connue, en un sel d'addition d'acide.

2. Procédé selon la revendication 1, caractérisé en ce que l'introduction du chlorure d'hydroxamoyle de formule (II) dans le solvant ou milieu de dispersion est effectuée à des températures de 10 à 25°C.

3. Procédé selon l'une des revendications 1 et/ou 2, caractérisé en ce qu'on ajoute une base, de préférence en la quantité molaire.

4. Procédé selon la revendication 3, caractérisé en ce qu'on ajoute, comme base, une amine secondaire ou tertiaire, un hydroxyde de métal alcalin, un carbonate de métal alcalin, un hydrogénocarbonate de métal alcalin ou un acétate de métal alcalin.

5. Procédé selon l'une des revendications 3 et 4, caractérisé en ce la base est ajoutée peu à peu ou par portions.

6. Procédé selon l'une quelconque des revendications 1 à 5, caractérisé en ce qu'on utilise, comme solvant, le diméthylformamide, le diméthylsulfoxyde, la N-méthylpyrrolidone, un alcool inférieur, le diéthyléther, l'eau ou un mélange de ces solvants.

7. Procédé selon l'une quelconque des revendications 1 à 6, caractérisé en ce que le radical R contient un radical —NH—.

8. Procédé selon l'une quelconque des revendications 1 à 7, caractérisé en ce que $R^4$ représente un radical $-(CH_2)_2-$ ou $-(CH_2)_3-$.

9. Procédé selon l'une quelconque des revendications 1 à 7, caractérisé en ce que $R^5$ représente un radical $-CH_2-$ ou $-(CH_2)_2-$.

10. Procédé selon l'une quelconque des revendications 1 à 7, caractérisé en ce que $R^2$ représente un radical méthyle ou éthyle.